# EUROPEAN PATENT SPECIFICATION

(11) **EP 3 580 721 B1**
(45) Date of publication and mention of the grant of the patent: **18.06.2025**
(21) Application number: 18706861.4
(22) Date of filing: 26.01.2018
(51) Int. Cl.: G06T 7/00, G06T 7/30

(54) **SYSTEM AND METHOD FOR PROCESSING DATA FROM COMPUTED TOMOGRAPHY SCANS**
SYSTEM UND VERFAHREN ZUR VERARBEITUNG VON DATEN AUS COMPUTERTOMOGRAFISCHEN SCANS
SYSTÈME ET PROCÉDÉ DE TRAITEMENT DE DONNÉES À PARTIR DE BALAYAGES DE TOMODENSITOMÉTRIE

(30) Priority: 10.02.2017 IT 201700014692
(43) Date of publication of application: 18.12.2019
(73) Proprietor: R.A.W. S.r.l., 20126 Milano (IT)
(72) Inventor: SOLBIATI, Marco, 21052 Busto Arsizio (Varese) (IT); PASSERA, Katia, 21052 Busto Arsizio (Varese) (IT); ROTILIO, Alessandro, 21052 Busto Arsizio (Varese) (IT)
(74) Representative: Penza, Giancarlo
(86) International application number: PCT/IB2018/050488
(87) International publication number: WO 2018/146573

(56) References cited:
- US-A1- 2008 033 420
- SORCINI B ET AL: "Clinical application of image-guided radiotherapy, IGRT (on the Varian OBI platform)", CANCER RADIOTHERAPIE, ELSEVIER, PARIS, FR, vol. 10, no. 5, 1 September 2006 (2006-09-01), pages 252 - 257, XP028033241, ISSN: 1278-3218, [retrieved on 20060901], DOI: 10.1016/J.CANRAD.2006.05.012
- MOUNTNEY PETER ET AL: "An Augmented Reality Framework for Soft Tissue Surgery", 14 September 2014, NETWORK AND PARALLEL COMPUTING; [LECTURE NOTES IN COMPUTER SCIENCE; LECT.NOTES COMPUTER], SPRINGER INTERNATIONAL PUBLISHING, CHAM, PAGE(S) 423 - 431, ISBN: 978-3-540-76785-5, ISSN: 0302-9743, XP047312307
- VEIGA CATARINA ET AL: "Toward adaptive radiotherapy for head and neck patients: Feasibility study on using CT-to-CBCT deformable registration for "dose of the day" calc", MEDICAL PHYSICS, AIP, MELVILLE, NY, US, vol. 41, no. 3, 1 March 2014 (2014-03-01), XP012182143, ISSN: 0094-2405, [retrieved on 19010101], DOI: 10.1118/1.4864240
- XIN ZHEN ET AL: "Paper;CT to cone-beam CT deformable registration with simultaneous intensity correction;CT to cone-beam CT deformable registration with simultaneous intensity correction", PHYSICS IN MEDICINE AND BIOLOGY, INSTITUTE OF PHYSICS PUBLISHING, BRISTOL GB, vol. 57, no. 21, 3 October 2012 (2012-10-03), pages 6807 - 6826, XP020230963, ISSN: 0031-9155, DOI: 10.1088/0031-9155/57/21/6807

## Description

### FIELD OF APPLICATION

The present invention relates to a system for processing data from computed axial tomography scans.

The present invention further relates to a method for processing data from computed axial tomography scans.

In particular, the present invention relates to a system and method for processing data from computed axial tomography scans relating to antitumour treatment methods.

More in particular, the present invention relates to a system and method for processing data from computed axial tomography scans relating to methods of antitumour treatment for the liver or lungs and the present description makes reference to this field of application to simplify the illustration thereof.

### PRIOR ART

At present, data from standard computed tomography (CT) scans prior to and after surgical treatment, for example by means of ablation of the tumour mass, are compared to establish whether a supplementary antitumour treatment is necessary.

Comparisons between the two standard computed tomography (CT) scans are point comparisons aimed at evaluating the one-to-one correspondence between pre-treatment image data representing a body area in which a tumour mass is present and post-treatment image data representing the same body area from which the tumour mass was treated.

Unfortunately, in order to be reliable, the processing described requires that the second standard computed tomography scan is performed no less than two weeks after the treatment.

This results in the need, in the event that the graphic processing does not confirm a perfect outcome of the treatment, for rehospitalization and a supplementary treatment, which will be followed by a new post-treatment standard computed tomography scan for comparison.

The overall processing of pre- and post-antitumour treatment data is therefore greatly delayed over time.

This obviously results in prolonged treatment periods and consequent health risks for a patient who is not promptly treated.

Furthermore, it is not feasible to perform a standard computed tomography scan intraoperatively at the end of the patient's antitumour treatment, so as to compare it with a pre-treatment computed tomography scan, because it would require excessively complex logistics and risks for the patient's health.

In light of the above, the processing of data from computed tomography scans for antitumour treatments is presently inefficient.

The object of the present invention is to provide an efficient system and method for processing data from computed tomography scans.

A particular object of the present invention is to provide a system and method for processing data from computed tomography scans that is efficient in terms of processing overall pre- and post-antitumour treatment data.

### SUMMARY OF THE INVENTION

In a first aspect of the invention, these and other objects are achieved by a system for processing data from computed tomography scans comprising: a first detection device capable of being activated prior to an antitumour treatment on a patient and that is configured to:
perform a standard computed tomography scan on a first part of a patient comprising a second part, which, in turn, comprises a tumour mass;
obtain, from said standard computed tomography scan, first image data, in a first reference system, comprising:
   a first portion of an image representing said first part of said patient;
   a second portion of an image representing said second part of said patient;
   a third portion of an image representing said tumour mass;
a second detection device capable of being activated intraoperatively at the end of the patient's antitumour treatment and configured to:
   perform a cone-beam computed tomography scan on a third part of said patient, wherein said third part substantially coincides with said second part and said third part comprises a necrotic area resulting from said antitumour treatment;
   obtain, from said cone-beam computed tomography scan, second image data, in a second reference system, comprising:
      a fourth portion an image representing said third part of said patient;
      a fifth portion an image representing said necrotic area;
   a processing unit, in data communication with said first detection device and said second detection device, and configured to process said first image data and said second image data, and which comprises:
      a first graphics processing module configured to carry out a first graphics processing step for graphically processing said first image data, thereby graphically highlighting, in said first image data, said second image portion and said third image portion;
      a second graphics processing module configured to carry out a second graphics processing step to make said first image data in said first reference system graphically comparable with said second image data in said second reference system;
      a third graphics processing module configured to graphically overlay said second image portion and said fourth image portion;
      a calculation module configured to calculate a degree of overlap between
         said third image portion, comprised in said second image portion and
         said fifth image portion comprised in said fourth image portion, thereby realizing a graphic comparison between said tumour mass prior to antitumour treatment and said necrotic area after antitumour treatment.

Advantageous aspects are disclosed in dependent claims 2 to 4.

The invention achieves a plurality of technical effects:
- efficient processing of data from computed tomography scans;
- in particular, efficient processing of data from computed tomography scans in terms of overall processing of data prior to and after antitumour treatment.
- consequent efficient antitumour treatment.

The technical effects/advantages cited and other technical effects/advantages of the invention will emerge in further detail from the description provided herein below of an example embodiment and it is provided by way of approximate and non-limiting example with reference to the attached drawings.

### BRIEF DESCRIPTION OF THE DRAWINGS

Figure 1 is a schematic description of the system/method of the invention.
Figures 2 to 5 show graphically processed images of the system/method of the invention.
Figure 6 is a general functional block diagram of the system of the invention.

### DETAILED DESCRIPTION

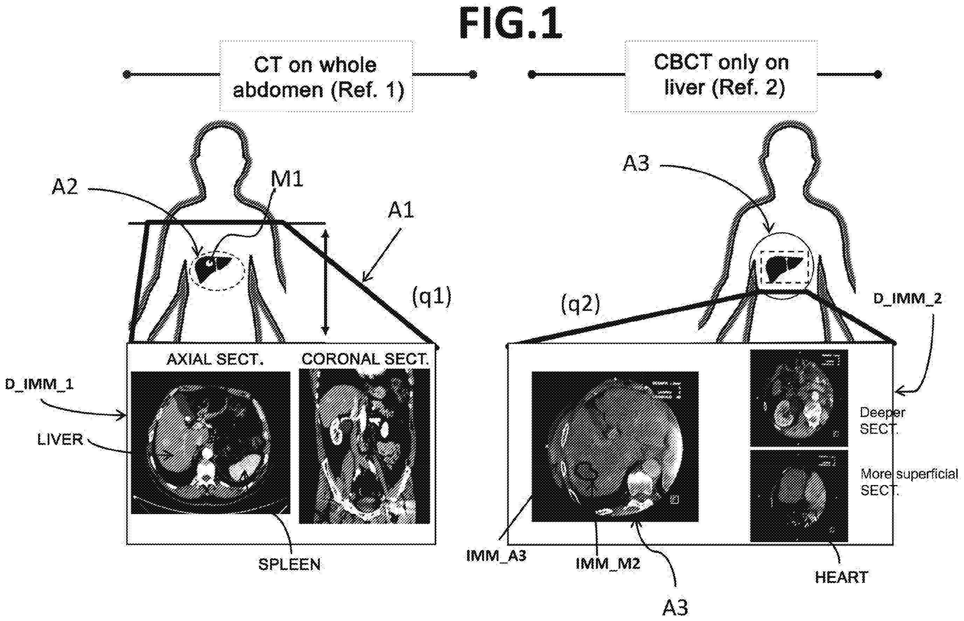

Figure 1 is a schematic description of the system/method of the invention, which schematically shows a comparison between a technique for a standard computed tomography scan focused on the whole abdomen of a patient with respective axial and coronal cross-sectional views (on the left in the figure), and a technique for a cone-beam computed tomography scan focused on the patient's liver with respective cross-sectional views on different scanning levels (on the right in the figure).

Tomography scans are performed prior to an antitumour treatment (standard computed tomography scan) and intraoperatively at the end of the patient's antitumour treatment (cone-beam computed tomography scan).

Tomography scans are performed by two different detection devices D1, D2 and the data produced are processed by a processing unit 100.

The antitumour treatment preferably takes place by means of ablation of a tumour mass, i.e. by destruction, in particular achieved with a heated needle, of neoplastic cells circulating in the body so as to prevent the development of metastases and recurrence.

In the present invention, the applicant has considered that the detail offered by a standard computed tomography scan is greater than that offered by a cone-beam computed tomography scan. Therefore, for diagnostic purposes, i.e. in order to diagnose a tumour, a standard computed tomography scan is used; in fact, it represents, in the case of a liver tumour, a scan of the whole abdomen (or more in general of the torso) and is therefore able to study the pathology also outside the liver (lymph nodes, etc); in the case of a lung tumour, on the other hand, it represents a scan of the torso and therefore it is possible to study the pathology also outside the lung.

The applicant has further considered that, also for tumour staging purposes, i.e. in order to describe schematically how large a tumour is and how far it has extended from the original site of development, a standard computed tomography scan is necessary.

The applicant has further considered that it is neither efficient nor useful to use a cone-beam computed tomography scan for the step of diagnosing a tumour, since a cone-beam computed tomography scan is only a focused scan of one part (for example the liver or a lung) and at times has difficulty in containing the entire part.

The applicant has further verified that the contrast medium used for a cone-beam computed tomography scan is in large doses (much larger compared to a standard computed tomography scan), so it is potentially harmful to consider giving two doses of contrast medium close together, i.e. it is harmful to consider doing a cone-beam computed tomography scan prior to treatment and intraoperatively at the end of the patient's antitumour treatment.

With reference to figure 1, the invention discloses a processing system and method for processing image data D_IMM_1, D_IMM2 from computed tomography scans, in particular from a standard computed tomography scan CT and from a cone-beam computed tomography scan CBCT.

The invention comprises performing a standard computed tomography scan CT on a first part A1 of a patient.

In a preferred embodiment of the invention, the first part A1 of the patient is an abdominal area, in particular comprising the liver plus the surrounding areas.

Alternatively, the first part A1 can be the area of the torso, i.e. the area of the abdomen and chest taken together.

The first part A1 comprises a second part A2 of a patient.

In a preferred embodiment of the invention, the second part A2 of the patient is the area of the liver.

In an alternative embodiment, the second part A2 of the patient is the area of a lung.

The second part A2 comprises, in turn, a tumour mass M1.

With reference to figure 1, the invention comprises obtaining, from the standard computed tomography scan CT, the first image data D_IMM_1, in a first reference system Rif_1.

According to the invention, the standard computed tomography scan CT is performed prior to an antitumour treatment on the patient, in particular on the tumour mass M1.

A first detection device (D1), capable of being activated prior to an antitumour treatment on the patient, is configured to perform a standard computed tomography scan CT on a first part A1 of the patient, which comprises a second part A2, which, in turn, comprises the tumour mass M1.

The first detection device (D1) is further configured to obtain, from the standard computed tomography scan CT, the first image data D_IMM_1, in the first reference system Rif_1.

In particular, the first reference system Rif_1 is a Cartesian coordinate system with three dimensions (X,Y,Z), characterized by a first predetermined image quality q1.

The standard computed tomography scan CT acquires a volume of interest and the 3D data are subsequently processed and rendered on three planes: axial, coronal and sagittal.

For example, with reference to figure 1, the first image data D_IMM_1, which comprise the axial and coronal cross sections of the first part A1 of the patient, are shown at the bottom left.

In particular, in the figure that shows the axial cross section, one can clearly see the liver (which occupies nearly the totality of the left part of the figure) and the spleen (at the bottom right in the figure).

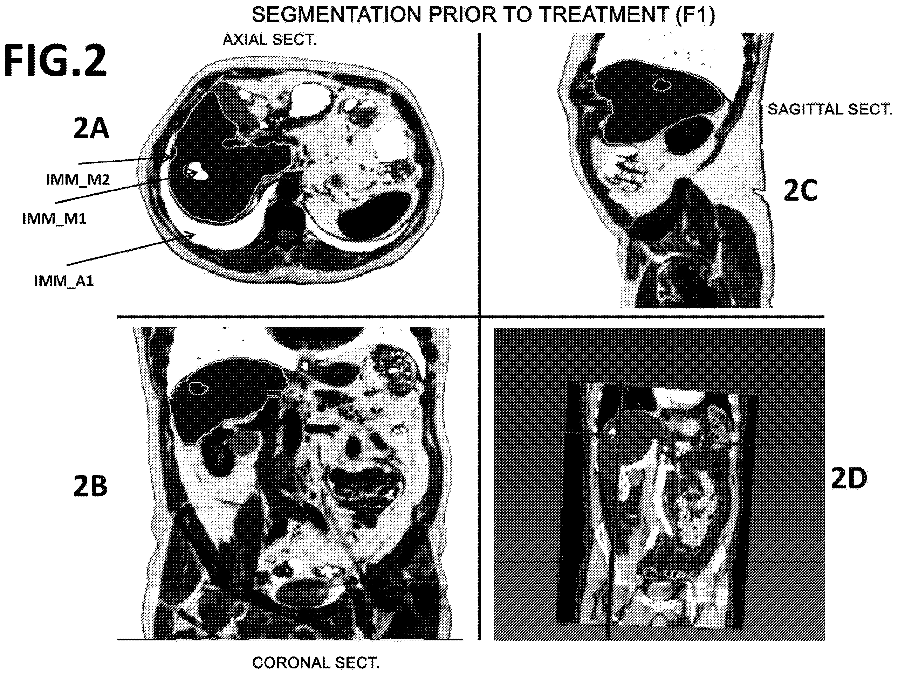

Figure 2 shows an example of graphically processed images; in particular, the images on the left represent the outcome of a standard computed tomography scan CT in axial and coronal cross sections and coincide with those of figure 1 at the bottom left.

With specific reference to figure 2, according to the invention, the first image data D_IMM_1 comprise:
- a first portion of an image IMM_A1 representing said first part A1 of said patient;
- a second portion of the image IMM_A2 representing said second part A2 of said patient;
- a third portion of the image IMM_M1 representing said tumour mass M1.

In particular, figure 2 shows a series of images obtained from the standard computed tomography scan CT prior to the antitumour treatment, according to an axial cross section 2A, a coronal cross section 2B, a sagittal cross section 2C and a cross section representing a window in which it is possible to move along three axes to inspect the volume of interest.

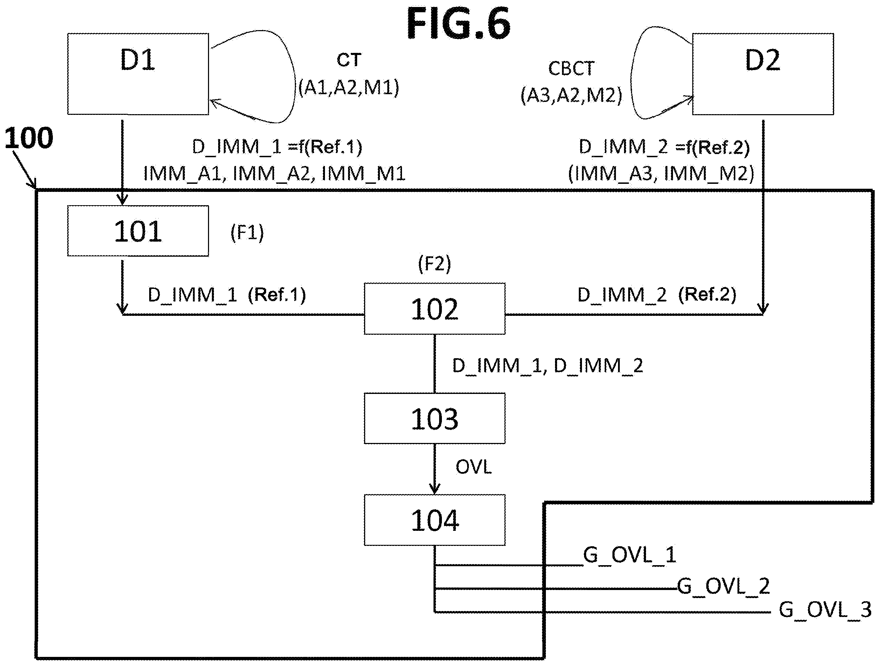

The invention comprises carrying out a first graphics processing step F1 (fig.6) for graphically processing the first image data D_IMM_1.

A technical effect of this processing is a graphic highlighting, in the first image data D_IMM_1, of the second image portion IMM_A2 and of the third image portion IMM_M1.

According to the invention, the first graphics processing step F1 is carried out by means of graphic segmentation of the first image data D_IMM_1.

In other words, with reference to figure 2, the first image data D_IMM_1 are partitioned into homogeneous regions on the basis of a certain criterion of belonging of the voxel (unit of volume) to a target region.

The technical effect is to identify/recognize the objects making up the image, so as to obtain a more compact representation for a detailed analysis of the images.

In other words, the segmentation enables the representation of the images to be simplified and/or changed into a more meaningful and/or easier-to-analyse graphic form.

The segmentation, carried out with reference to figure 2, enables precise identification of the area of the cross sections 2A, 2B, 2C and 2D, in which the second part A2 represented by the second image portion IMM_A2, in particular the patient's liver, is present, and in which the tumour mass M1 represented by the third image portion IMM_M1 representing the tumour mass M1 is present.

Following the described segmentation prior to the antitumour treatment, the patient undergoes an antitumour treatment intraoperatively.

The antitumour treatment preferably takes place by means of ablation of the tumour mass M1, i.e. by means of destruction, in particular achieved with a heated needle, of neoplastic cells circulating in the body so as to prevent the development of metastases and recurrence.

Intraoperatively, at the end of the patient's antitumour treatment,
the invention comprises performing a cone-beam computed tomography scan CBCT on a third part A3 of the patient.

In a preferred embodiment of the invention, the third part A3 substantially coincides with the second part A2, in particular the patient's liver. Alternatively, the third part A3, which substantially coincides with the second part A2, is a lung of the patient.

In a preferred embodiment of the invention, the third part A3 comprises a necrotic area M2 resulting from the antitumour treatment.

The invention comprises obtaining, from the cone-beam computed tomography scan CBCT, the second image data D_IMM_2 in a second reference system Rif_2 (fig. 1).

According to the invention, the cone-beam computed tomography scan CBCT is performed intraoperatively at the end of the patient's antitumour treatment.

A second detection device D2 capable of being activated intraoperatively at the end of the patient's antitumour treatment is configured to perform the cone-beam computed tomography scan CBCT on the third part A3 of the patient, which substantially coincides with the second part A2, and comprises the necrotic area M2 resulting from said antitumour treatment. A second detection device D2 is further configured to obtain, from the cone-beam computed tomography scan CBCT, the second image data D_IMM_2 in a second reference system Rif_2.

In particular, as schematically shown in figure 1 on the right, the second reference system Rif_2 is a Cartesian coordinate system with three dimensional cone-beam coordinates X,Y,Z characterized by a second predetermined image quality q2 of a lesser quality than the first predetermined image quality q1.

With specific reference to figure 1, according to the invention, the second image data D_IMM_2 comprise:
- a fourth portion of the image IMM_A3 representing the third part A3 of the patient;
- a fifth portion of the image IMM_M2 representing the necrotic area M2.

In particular, figure 1 shows a series of images obtained from the cone-beam computed tomography scan CBCT at the end of the patient's antitumour treatment, according to axial cross sections at a different scanning level.

As already mentioned, the invention comprises a processing unit 100, in particular with reference to figure 6, in data communication with the first detection device (D1) and the second detection device D2, and configured to process the first image data D_IMM_1 and the second image data D_IMM_2.

In general, it should be noted that in the present context and in the subsequent claims, the processing unit 100 is presented as being split into distinct functional modules (storage modules and operative modules) for the sole purpose of describing its functionalities clearly and completely.

The processing unit can consist of a single electronic device, appropriately programmed to perform the functionalities described, and the different modules can correspond to hardware entities and/or routine software that are part of the programmed device.

Alternatively or additionally, these functions can be performed by a plurality of electronic devices in which the above-mentioned functional modules can be distributed.

The processing unit 100 can also make use of one or more processors for execution of the instructions contained in the memory modules.

The above-mentioned functional modules can also be distributed in different computers, locally or remotely, based on the architecture of the network in which they reside.

According to the invention, the processing unit 100 comprises a first graphics processing module 101 configured to perform the first step F1 (fig.2) for graphically processing the first image data D_IMM_1, thereby graphically highlighting, in the first image data D_IMM_1, the second image portion IMM_A2 and the third image portion IMM_M1.

In other words, the first graphics processing module 101 carries out the graphic segmentation of the first image data D_IMM_1, previously described.

The invention comprises performing a second graphics processing step F2 (fig. 6) between the first reference system Rif_1 and the second reference system Rif_2.

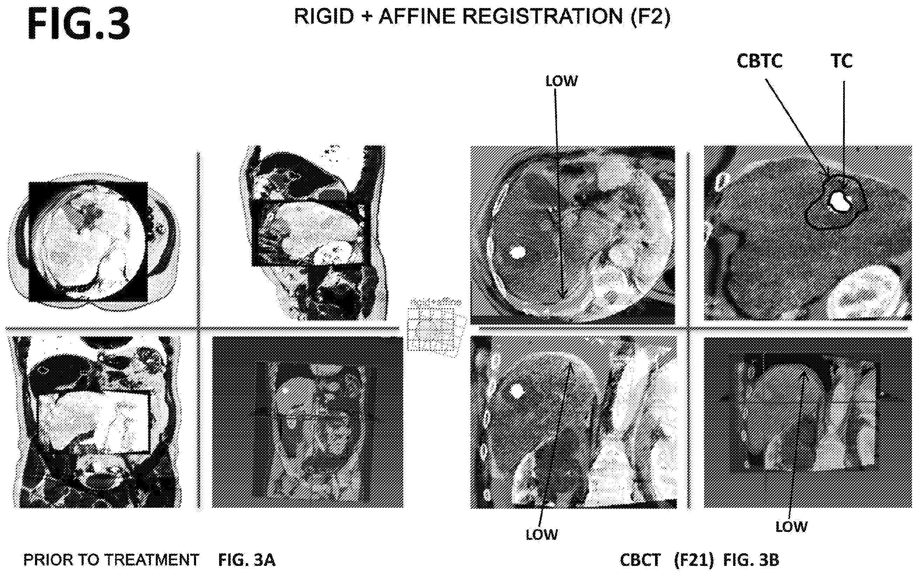

Figure 3A shows a comparison between the initial state of the standard computed tomography scan CT, represented by the cross sections almost in their entirety, and the initial state of the cone-beam computed tomography scan CBCT, represented by the area inside the white line; as can be seen, prior to the graphics processing step F2, the two tomography scans are not comparable.

The technical effect achieved by the second graphics processing step F2 is thus to make the first image data D_IMM_1 and second image data D_IMM_2 graphically comparable.

For this purpose, the processing unit 100 comprises a second graphics processing module (102) configured to carry out a second graphics processing step F2 to make the first image data D_IMM_1 in the first reference system Rif_1 graphically comparable with the second image data D_IMM_2 in the second reference system Rif_2.

The second graphics processing step F2 is carried out by means of graphic registration of the first image data D_IMM_1, obtained with the standard computed tomography scan CT, with the second image data D_IMM_2 obtained with the cone-beam computed tomography scan CBCT.

According to the invention, with reference to figure 3 (images on the right), the graphic registration comprises a step F21 of rigid registration and affine registration.

The second graphics processing module 102 is configured to carry out the step of rigid registration and affine registration F21.

The technical effect is to ensure a generic graphic realignment of the first image data D_IMM_1 with the second image data D_IMM_2, wherein the realignment comprises rotation, translation and relative scaling (contraction or expansion) of the first and second image data.

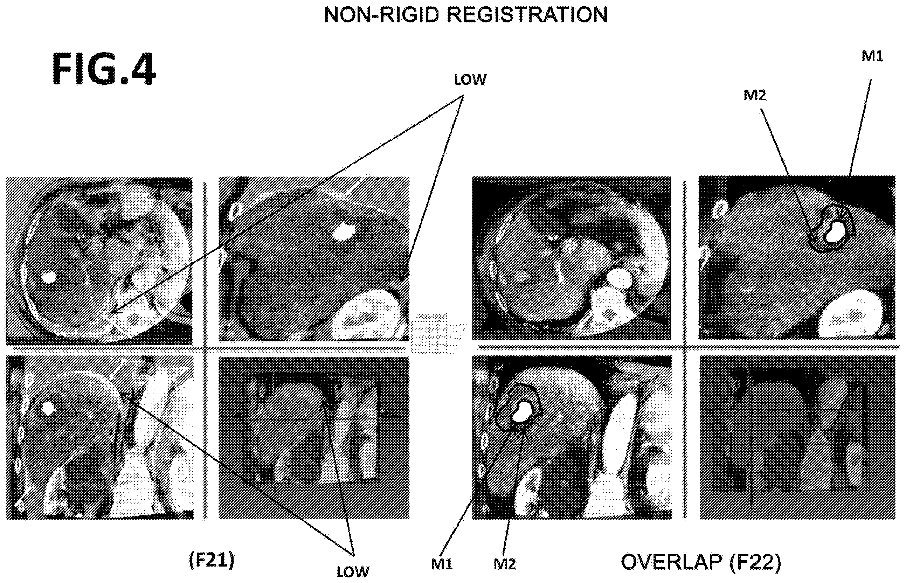

The effect is shown in figure 3 (or fig. 4 in the group of images on the left), where, however, one may note low definition shadowy areas LOW where the rigid and affine registration was not able to perfectly register the first and second image data, which are not perfectly aligned.

Consequently, the "visibility" of such areas is limited, which can represent a large difficulty in identifying a possible tumour mass.

According to the invention, with reference to figure 4, the graphic registration further comprises a step of non-rigid registration F22.

The second graphics processing module 102 is configured to perform the non-rigid registration step F22.

The technical effect is to ensure that the first image data D_IMM_1 and the second image data D_IMM_2 are realigned so as to correct local deformations.

In other words, the non-rigid registration step F22 enables the shadowy areas LOW to be eliminated by aligning the first image data D_IMM_1 and second image data D_IMM_2, thereby also correcting deformations that are only local and do not regard the whole image; this enables maximum "visibility" of the area affected by a possible tumour.

The technical effect can be seen in figure 4 (group of images on the right), where it is evident that the shadowy areas LOW are no longer present.

At the end of the graphics processing step F2, which achieves a graphic registration of the first image data D_IMM_1 with the second image data D_IMM_2, the image data are comparable for subsequent processing.

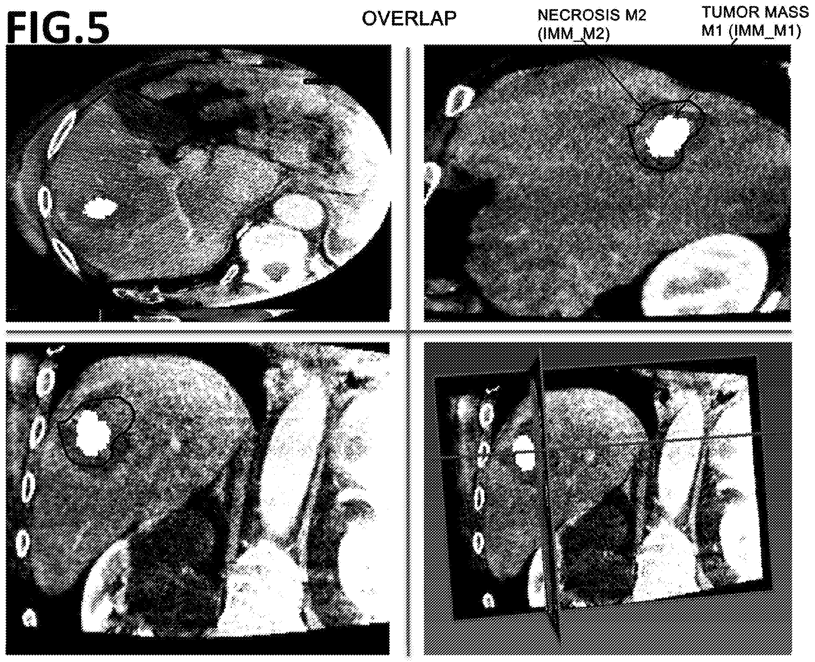

With reference to figure 5, the invention comprises graphically overlaying the second image portion IMM_A2 on the fourth image portion IMM_A3.

According to the invention, a third graphics processing module 103 of the processing unit 100 is configured to graphically overlay OVL the second image portion IMM_A2 and the fourth image portion IMM_A3.

In other words, in a preferred embodiment, the overlaying step comprises overlaying the image of the liver IMM_A2 produced with the standard computed tomography scan CT with the image of the liver IMM_A3 produced with the cone-beam computed tomography scan CBCT.

In an alternative embodiment, the overlaying step comprises overlaying the image of a lung IMM_A2 produced with the standard computed tomography scan CT with the image of a lung IMM_A3 produced with the cone-beam computed tomography scan CBCT.

Advantageously, the invention comprises calculating the degree of overlap G_OVL between the third image portion IMM_M1, comprised in the second image portion IMM_A2 and the fifth image portion IMM_M2 comprised in the fourth image portion IMM_A3.

A calculation module 104 of the processing unit 100 is configured to calculate the degree of overlap G_OVL.

In other words, the invention comprises making a graphic comparison between the tumour mass M1 prior to the antitumour treatment and the necrotic area M2 after the antitumour treatment.

The technical effect achieved is an evaluation of a correspondence between the limits of the tumour prior to treatment and the limits of the area that was treated, preferably by ablation, in the intraoperative phase.

According to the invention, the step of calculating the degree of overlap G_OVL determines one of the following conditions:
- complete overlap G_OVL1 representing an antitumour treatment carried out successfully
- partial overlap G_OVL2 representing the need to consider the advisability of further intraoperative antitumour treatment;
- absence of overlap G_OVL3 representing the need for further intraoperative antitumour treatment.

The calculation module 104 (fig.6) is configured to calculate the degree of overlap G_OVL and determine one of the above-mentioned conditions.

The graphic registration of the invention enables the tumour prior to treatment to be compared with ablation after treatment (or during treatment, given that it is a CBCT scan), in particular in the liver or in a lung; the comparison is made on the basis of graphic processing.

The retreatment can thus be performed directly at the same operating site if it is seen that, after the treatment, the tumour area was not completely centered or was only partially centered.

Up to now, the comparison was made by performing a standard computed tomography scan the day after the treatment, and it was necessary to rehospitalize the patient and do another session in the operating room, in the event that the treatment was unsuccessful.

The method/system of the invention is thus highly efficient and protects the patient's health.

## Claims

1. A processing system for processing image data (D_IMM_1, D_IMM2) from computed tomography scans (CT, CBCT) in antitumour treatment of ablation of a tumour mass, comprising:
a first detection device (D1) capable of being activated prior to an ablation antitumour treatment on a patient and that is configured to:
- perform a standard computed tomography scan (CT) on a first part (A1) of a patient, said first part (A1) comprising a second part (A2), which, in turn, comprises a tumour mass (M1);
- obtain, from said standard computed tomography scan (CT), first image data (D_IMM_1), in a first reference system (Rif_1), comprising:
• a first portion of an image (IMM_A1) representing said first part (A1) of said patient;
• a second portion of the image (IMM_A2) representing said second part (A2) of said patient;
• a third portion of the image (IMM_M1) representing said tumour mass (M1);
a second detection device (D2) capable of being activated intraoperatively at the end of the patient's ablation antitumour treatment and that is configured to:
- perform a cone-beam computed tomography scan (CBCT) on a third part (A3) of said patient, wherein said third part (A3) substantially coincides with said second part (A2) and said third part (A3) comprises a necrotic area (M2) resulting from said ablation antitumour treatment;
- obtain, from said cone-beam computed tomography scan (CBCT), second image data (D_IMM_2) in a second reference system (Rif_2), said second image data (D_IMM_2) comprising:
• a fourth portion of the image (IMM_A3) representing said third part (A3) of said patient;
• a fifth portion of the image (IMM_M2) representing said necrotic area (M2);
a processing unit (100), in data communication with said first detection device (D1) and said second detection device (D2), and configured to process said first image data (D_IMM_1) and said second image data (D_IMM_2), and which comprises:
- a first graphics processing module (101) configured to carry out a first graphics processing step (F1) for graphically processing said first image data (D_IMM_1) by means of graphic segmentation of said first image data (D_IMM_1), thereby graphically highlighting, in said first image data (D_IMM_1), said second image portion (IMM_A2) and said third image portion (IMM_M1);
- a second graphics processing module (102) configured to carry out a second graphics processing step (F2),
by means of graphic registration of:
- said first image data (D_IMM_1), obtained with said standard computed tomography scan (CT) with
- said second image data (D_IMM_2), obtained with said cone-beam computed tomography scan (CBCT),
to make said first image data (D_IMM_1) in said first reference system (Rif_1) graphically comparable with said second image data (D_IMM_2) in said second reference system (Rif_2);
- a third graphics processing module (103) configured to graphically overlay (OVL) said second image portion (IMM_A2) and said fourth image portion (IMM_A3);
- a calculation module (104) configured to calculate a degree of overlap (G_OVL) between:
• said third image portion (IMM_M1), comprised in said second image portion (IMM_A2) and
• said fifth image portion (IMM_M2) comprised in said fourth image portion (IMM_A3),
thereby realizing a graphic comparison between said tumour mass (M1) prior to ablation antitumour treatment and said necrotic area (M2) after ablation antitumour treatment.

2. The processing system according to either claim 1, wherein said calculation module (104) is configured to calculate the degree of overlap (G_OVL) and to determine one of the following conditions:
- complete overlap (G_OVL1) representing an ablation antitumour treatment carried out successfully;
- partial overlap (G_OVL2) representing the need to consider the advisability of further intraoperative ablation antitumour treatment;
- absence of overlap (G_OVL3) representing the need for further intraoperative ablation antitumour treatment.

3. The processing system according to claim 1, wherein said second graphics processing module (102) is configured to carry out, in said graphic registration:
- a step of rigid registration and affine registration (F21), thereby performing a generic graphic realignment of said first image data (D_IMM_1) with said second image data (D_IMM_2), said realignment comprising rotation, translation and relative scaling of the first and second image data;
- a step of non-rigid registration (F22), thereby performing a precise graphic realignment of said first image data (D_IMM_1) with said second image data (D_IMM_2), so as to correct local deformations.

4. The system according to any one of the preceding claims, wherein said first part (A1) of the patient is an area of the torso comprising said second part (A2), wherein said second part (A2) is the area of the liver or of a lung, and said third part (A3) substantially coincides with said second part (A2).

## Patentansprüche

1. Verarbeitungssystem zur Verarbeitung von Bilddaten (D_IMM_1, D_IMM2) aus computertomografischen Scans (CT, CBCT) bei der Antitumorbehandlung der Ablation einer Tumormasse, umfassend:
eine erste Detektionsvorrichtung (D1), die vor einer Ablationsantitumorbehandlung bei einem Patienten aktiviert werden kann und die dazu ausgelegt ist:
- einen computertomografischen Standard-Scan (CT) an einem ersten Teil (A1) eines Patienten durchzuführen, wobei der erste Teil (A1) einen zweiten Teil (A2) umfasst, der wiederum eine Tumormasse (M1) umfasst;
- erste Bilddaten (D_IMM_1) aus dem computertomografischen Standard-Scan (CT) in einem ersten Referenzsystem (Rif_1) zu erhalten, umfassend:
• einen ersten Abschnitt eines Bildes (IMM_A1), der den ersten Teil (A1) des Patienten darstellt;
• einen zweiten Abschnitt des Bildes (IMM_A2), der den zweiten Teil (A2) des Patienten darstellt;
• einen dritten Abschnitt des Bildes (IMM_M1), der die Tumormasse (M1) darstellt;
eine zweite Detektionsvorrichtung (D2), die am Ende der Ablationsantitumorbehandlung des Patienten intraoperativ aktiviert werden kann und die dazu ausgelegt ist:
- einen computertomografischen Kegelstrahl-Scan (CBCT) an einem dritten Teil (A3) des Patienten durchzuführen, wobei der dritte Teil (A3) im Wesentlichen mit dem zweiten Teil (A2) zusammenfällt und der dritte Teil (A3) einen nekrotischen Bereich (M2) umfasst, der sich aus der Ablationsantitumorbehandlung ergibt;
- zweite Bilddaten (D_IMM_2) aus dem computertomografischen Kegelstrahl-Scan (CBCT) in einem zweiten Referenzsystem (Rif_2) zu erhalten, wobei die zweiten Bilddaten (D_IMM_2) Folgendes umfassen:
• einen vierten Abschnitt des Bildes (IMM_A3), der den dritten Teil (A3) des Patienten darstellt;
• einen fünften Abschnitt des Bildes (IMM_M2), der den nekrotischen Bereich (M2) darstellt;
eine Verarbeitungseinheit (100), die in Datenkommunikation mit der ersten Detektionsvorrichtung (D1) und der zweiten Detektionsvorrichtung (D2) steht und dazu ausgelegt ist, die ersten Bilddaten (D_IMM_1) und die zweiten Bilddaten (D_IMM_2) zu verarbeiten, und
die Folgendes umfasst:
- ein erstes Grafikverarbeitungsmodul (101), das dazu ausgelegt ist, einen ersten Grafikverarbeitungsschritt (F1) zum grafischen Verarbeiten der ersten Bilddaten (D_IMM_1) mittels grafischer Segmentierung der ersten Bilddaten (D_IMM_1) auszuführen, wodurch der zweite Bildabschnitt (IMM_A2) und der dritte Bildabschnitt (IMM_M1) in den ersten Bilddaten (D_IMM_1) grafisch hervorgehoben werden;
- ein zweites Grafikverarbeitungsmodul (102), das dazu ausgelegt ist, einen zweiten Grafikverarbeitungsschritt (F2) auszuführen, mittels grafischer Registrierung von:
- den ersten Bilddaten (D_IMM_1), erhalten mit dem computertomografischen Standard-Scan (CT) mit
- den zweiten Bilddaten (D_IMM_2), erhalten mit dem computertomografischen Kegelstrahl-Scan (CBCT), um die ersten Bilddaten (D_IMM_1) in dem ersten Referenzsystem (Rif_1) grafisch mit den zweiten Bilddaten (D_IMM_2) in dem zweiten Referenzsystem (Rif_2) vergleichbar zu machen;
- ein drittes Grafikverarbeitungsmodul (103), das dazu ausgelegt ist, den zweiten Bildabschnitt (IMM_A2) und den vierten Bildabschnitt (IMM_A3) grafisch zu überlagern (OVL);
- ein Berechnungsmodul (104), das dazu ausgelegt ist, einen Grad der Überlappung (G_OVL) zwischen Folgendem zu berechnen:
• dem dritten Bildabschnitt (IMM_M1), der in dem zweiten Bildabschnitt (IMM_A2) enthalten ist, und
• dem fünften Bildabschnitt (IMM_M2), der in dem vierten Bildabschnitt (IMM_A3) enthalten ist,
wodurch ein grafischer Vergleich zwischen der Tumormasse (M1) vor der Ablationsantitumorbehandlung und dem nekrotischen Bereich (M2) nach der Ablationsantitumorbehandlung realisiert wird.

2. Verarbeitungssystem nach Anspruch 1, wobei das Berechnungsmodul (104) dazu ausgelegt ist, den Grad der Überlappung (G_OVL) zu berechnen und eine der folgenden Bedingungen zu bestimmen:
- vollständige Überlappung (G_OVL1), die eine erfolgreich ausgeführte Ablationsantitumorbehandlung darstellt;
- partielle Überlappung (G_OVL2), die die Notwendigkeit darstellt, zu prüfen, ob eine weitere intraoperative Ablationsantitumorbehandlung ratsam wäre;
- Fehlen einer Überlappung (G_OVL3), die die Notwendigkeit einer weiteren intraoperativen Ablationsantitumorbehandlung darstellt.

3. Verarbeitungssystem nach Anspruch 1, wobei das zweite Grafikverarbeitungsmodul (102) ausgelegt ist, um in der Grafikregistrierung auszuführen:
- einen Schritt zur starren Registrierung und affinen Registrierung (F21), wodurch eine generische grafische Neuausrichtung der ersten Bilddaten (D_IMM_1) mit den zweiten Bilddaten (D_IMM_2) durchgeführt wird, wobei die Neuausrichtung Drehung, Translation und relative Skalierung der ersten und zweiten Bilddaten umfasst;
- einen Schritt zur nicht starren Registrierung (F22), wodurch eine präzise grafische Neuausrichtung der ersten Bilddaten (D_IMM_1) mit den zweiten Bilddaten (D_IMM_2) durchgeführt wird, um lokale Verformungen zu korrigieren.

4. System nach einem der vorhergehenden Ansprüche, wobei der erste Teil (A1) des Patienten ein Bereich des Rumpfes ist, der den zweiten Teil (A2) umfasst, wobei der zweite Teil (A2) der Bereich der Leber oder einer Lunge ist und der dritte Teil (A3) im Wesentlichen mit dem zweiten Teil (A2) zusammenfällt.

## Revendications

1. Système de traitement destiné au traitement de données d'image (D_IMM_1, D_IMM2) à partir de balayages de tomodensitométrie (CT, CBCT) dans le cadre d'un traitement antitumoral par ablation d'une masse tumorale, comprenant :
un premier dispositif de détection (D1) capable d'être activé avant un traitement antitumoral par ablation sur un patient et qui est configuré pour :
- réaliser un balayage de tomodensitométrie (CT) standard sur une première partie (A1) d'un patient, ladite première partie (A1) comprenant une deuxième partie (A2) qui, à son tour, comprend une masse tumorale (M1) ;
- obtenir, à partir dudit balayage de tomodensitométrie (CT) standard, des premières données d'image (D_IMM_1), dans un premier système de référence (Rif_1), comprenant :
• une première partie d'une image (IMM_A1) représentant ladite première partie (A1) dudit patient ;
• une deuxième partie de l'image (IMM_A2) représentant ladite deuxième partie (A2) dudit patient ;
• une troisième partie de l'image (IMM_M1) représentant ladite masse tumorale (M1) ;
un second dispositif de détection (D2) capable d'être activé de manière peropératoire à la fin du traitement antitumoral par ablation du patient et qui est configuré pour :
- réaliser un balayage de tomodensitométrie à faisceau conique (CBCT) sur une troisième partie (A3) dudit patient, dans lequel ladite troisième partie (A3) coïncide sensiblement avec ladite deuxième partie (A2) et ladite troisième partie (A3) comprend une zone nécrotique (M2) résultant dudit traitement antitumoral par ablation ;
- obtenir, à partir dudit balayage de tomodensitométrie à faisceau conique (CBCT), des deuxièmes données d'images (D_IMM_2) dans un second système de référence (Rif_2), lesdites deuxièmes données d'images (D_IMM_2) comprenant :
• une quatrième partie de l'image (IMM_A3) représentant ladite troisième partie (A3) dudit patient ;
• une cinquième partie de l'image (IMM_M2) représentant ladite zone nécrotique (M2) ;
une unité de traitement (100), en communication de données avec ledit premier dispositif de détection (D1) et ledit second dispositif de détection (D2), et
configurée pour traiter lesdites premières données d'image (D_IMM_1) et lesdites deuxièmes données d'image (D_IMM_2), et qui comprend :
- un premier module de traitement graphique (101) configuré pour mettre en œuvre une première étape de traitement graphique (F1) pour traiter graphiquement lesdites premières données d'image (D_IMM_1) au moyen d'une segmentation graphique desdites premières données d'image (D_IMM_1), mettant ainsi en évidence graphiquement, dans lesdites premières données d'image (D_IMM_1), ladite deuxième partie d'image (IMM_A2) et ladite troisième partie d'image (IMM_M1) ;
- un deuxième module de traitement graphique (102) configuré pour mettre en œuvre une seconde étape de traitement graphique (F2), au moyen d'un enregistrement graphique :
- desdites premières données d'image (D_IMM_1), obtenues par ledit balayage de tomodensitométrie (CT) standard avec
- lesdites deuxièmes données d'image (D_IMM_2), obtenues par ledit balayage de tomodensitométrie à faisceau conique (CBCT), pour rendre lesdites premières données d'image (D_IMM_1) dans ledit premier système de référence (Rif_1) graphiquement comparables avec lesdites deuxièmes données d'image (D_IMM_2) dans ledit second système de référence (Rif_2) ;
- un troisième module de traitement graphique (103) configuré pour superposer graphiquement (OVL) ladite deuxième partie d'image (IMM_A2) et ladite quatrième partie d'image (IMM_A3) ;
- un module de calcul (104) configuré pour calculer un degré de superposition (G_OVL) entre :
• ladite troisième partie d'image (IMM_M1), comprise dans ladite deuxième partie d'image (IMM_A2) et
• ladite cinquième partie d'image (IMM_M2) comprise dans ladite quatrième partie d'image (IMM_A3),
réalisant ainsi une comparaison graphique entre ladite masse tumorale (M1) avant traitement antitumoral par ablation et ladite zone nécrotique (M2) après traitement antitumoral par ablation.

2. Système de traitement selon la revendication 1, dans lequel ledit module de calcul (104) est configuré pour calculer le degré de superposition (G_OVL) et pour déterminer l'une des conditions suivantes :
- un chevauchement complet (G_OVL1) représentant un traitement antitumoral par ablation réalisé avec succès ;
- un chevauchement partiel (G_OVL2) représentant la nécessité de considérer l'opportunité d'un traitement antitumoral par ablation peropératoire supplémentaire ;
- une absence de chevauchement (G_OVL3) représentant la nécessité d'un traitement antitumoral par ablation peropératoire supplémentaire.

3. Système de traitement selon la revendication 1, dans lequel ledit deuxième module de traitement graphique (102) est configuré pour effectuer, dans ledit enregistrement graphique :
- une étape d'enregistrement rigide et d'enregistrement affine (F21), réalisant ainsi un réalignement graphique générique desdites premières données d'image (D_IMM_1) avec lesdites deuxièmes données d'image (D_IMM_2), ledit réalignement comprenant la rotation, la translation et la mise à l'échelle relative des premières et deuxièmes données d'image ;
- une étape d'enregistrement non-rigide (F22), réalisant ainsi un réalignement graphique précis desdites premières données d'image (D_IMM_1) avec lesdites deuxièmes données d'image (D_IMM_2), de manière à corriger les déformations locales.

4. Système selon l'une quelconque des revendications précédentes, dans lequel ladite première partie (A1) du patient est une zone du torse comprenant ladite deuxième partie (A2), dans lequel ladite deuxième partie (A2) est la zone du foie ou d'un poumon, et ladite troisième partie (A3) coïncide sensiblement avec ladite deuxième partie (A2).
